# EUROPEAN PATENT APPLICATION

(11) **EP 4 534 526 A1**
(43) Date of publication of application: **09.04.2025**
(21) Application number: 23201561.0
(22) Date of filing: 04.10.2023
(51) Int. Cl.: C07C 231/12, C07C 231/14, C07C 237/46

(54) **OPTIMIZED PROCESS FOR THE PREPARATION OF IOPAMIDOL**

(71) Applicant: Bracco Imaging SPA, 20134 Milan (IT)
(72) Inventor: BUONSANTI, Federica, 10010 Colleretto Giacosa (IT); MAZZON, Roberta, 10010 Colleretto Giacosa (IT); FRETTA, Roberta, 10010 Colleretto Giacosa (IT)
(74) Representative: Ravizza, Claudio

(57) **Abstract**

The present invention relates to the field of organic chemistry, in particular to the synthesis of iodinated contrast agents: More in particular, the invention relates to a sustainable process for the synthesis of iopamidol which allows to improve the purity profile of the product and avoids the use of toxic reagents and solvents.

## Description

### Field of the invention

The present invention relates to the field of organic chemistry, in particular to the synthesis of iodinated contrast agents. More in particular, the invention relates to a sustainable and reliable process for the synthesis of iopamidol which allows to improve the purity profile of the product and avoids the use of toxic reagents and solvents.

### Background of the invention

Iodinated contrast agents are well-known compounds widely used in X-ray imaging diagnostic techniques. Among these compounds, S-(*N*,*N'*-bis[2-hydroxy-1-(hydroxymethyl)ethyl]-5-[[(2S)-2-hydroxy-1-oxopropyl]-amino]-2,4,6,triiodo-1,3-benzene dicarboxamide (iopamidol) is one of the numerous tri-iodinated radiographic contrast agent commercially available and widely used in daily diagnostic investigations by X-ray (The Merck Index, RSC Publishing, 15th Ed., 2013, 940-941; Lusic, H. et al., Chem. Rev. 2013, 113, 1641-1666).

The widespread use of this compound in diagnostic imaging makes necessary for the manufacturers to dispose of easy and convenient syntheses suitable for an industrial scale. This compound can be prepared by using a synthetic procedure known since the eighties and disclosed e.g. in GB 1,472,050. An industrial synthesis of iopamidol, characterized by the conversion of aromatic amino derivatives into the corresponding carboxamides by reaction with a suitable α-hydroxyacid derivative, is shown in the following Scheme 1 and described, for example, in US 4,001,323.

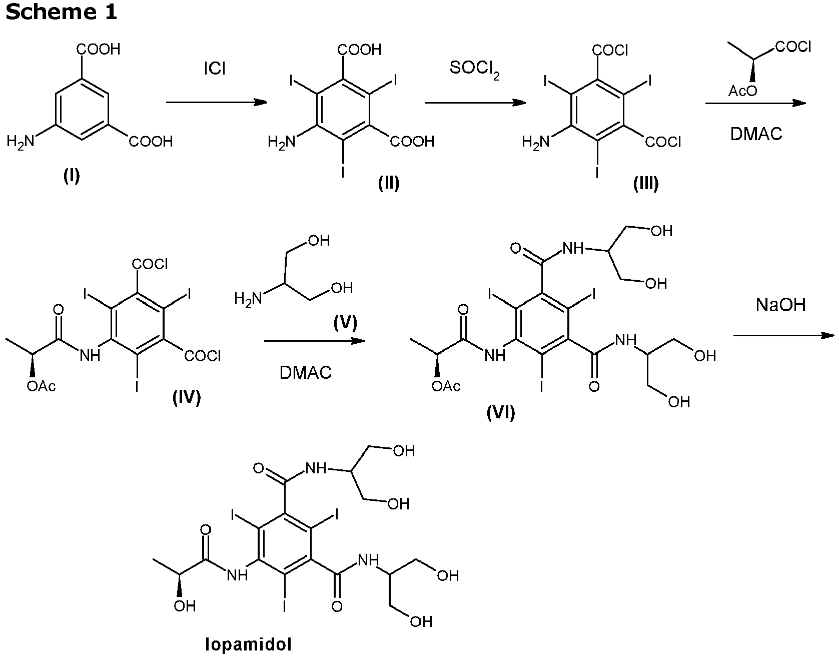

Several alternative synthetic approaches for preparing iopamidol were subsequently developed in order to optimize the different steps of the production. For instance, an alternative synthesis was described in patent application WO2002/044125 (see the following Scheme 2), providing an one-pot process with only isolation of the key intermediate 5-amino-*N,N'*-bis[2-hydroxy-1-(hydroxymethyl)ethyl]-2,4,6-triiodo-1,3-benzenedicarboxamide (IX).

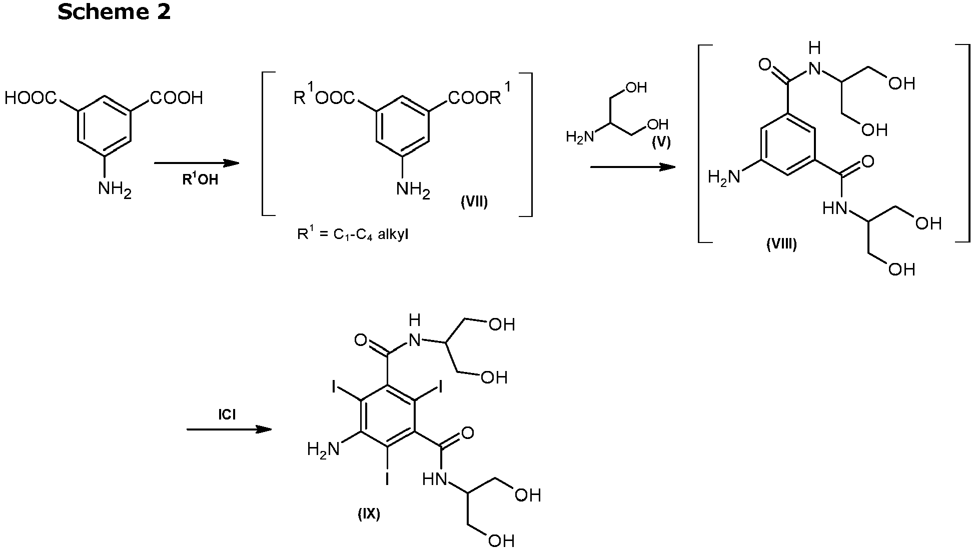

This approach allows to increase the overall yields, while avoiding the use of toxic SOCl₂ that was required for the preparation of the chloro-derivative (III) and reducing the amount of the correlated chlorinated impurities in the final iopamidol product. The synthesis described in WO2002/044125 proceeds with the protection of the diol groups using a large excess of acetic anhydride and the acetylation of the corresponding intermediate. However, this excessive use of acetic anhydride represents a drawback of this approach because its presence in the mixture, even as a residual, is detrimental for the next reaction steps.

A more efficient approach for the conversion of intermediate (IX) into iopamidol was described in WO2015/067601 (see the following Scheme 3), providing a one-pot process, with the isolation of intermediate (IX) only, and using a boric acid derivative as diol protecting group, which can be conveniently removed and recovered by extraction and re-used in the process, making it more efficient and sustainable.

Moreover, the first part of the synthesis is started from 5-nitro-isophthalic acid (5-NIPA) instead of the 5-amino derivative, strongly reducing the amount of methanesulfonic acid (MSA) necessary to catalyze the esterification step.

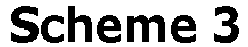

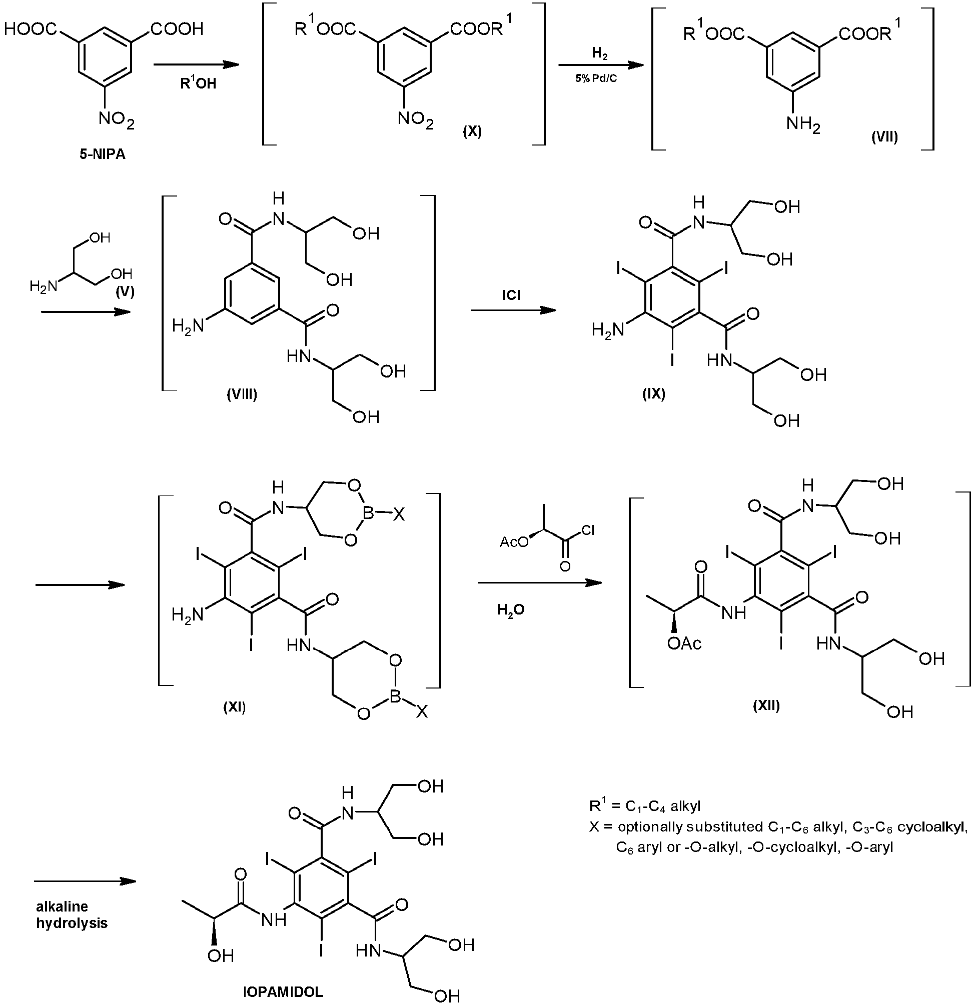

The intermediate (XI), having the diol groups linked to a boron protecting agent is reacted with (S)-2-(acetyloxy)propanoyl chloride to provide the N-(S)-2-(acetyloxy)propanoyl derivative (XII). Such reaction is carried out in a suitable medium selected among the dipolar aprotic solvents, preferably *N*,*N*-dimethylacetamide (DMAC), which provide the best performance in terms of solubilization of the starting materials and yield of the intermediate (XII).

The use of DMAC however could represent an issue, since it is flagged under the "Registration, Evaluation, Authorisation and restriction of Chemicals" (REACH) legislation as Substance of Very High Concern, especially when it is used in the bulk volumes of an industrial production. In fact, the regulators are more frequently inviting to the use of more sustainable and environmentally friendly solutions when performing chemical reactions in the pharmaceutical industry, so that new alternative solutions are being evaluated and tested in this direction, in particular with the aim of reducing or eliminating the use of DMAC or other similar substances. Moreover, avoiding the use of toxic materials not only decreases potential hazards, but can also reduce the overall costs of a process for the waste disposal and the like. Furthermore, the use of DMAC in the above-mentioned processes gives rise to a certain amount of the by-product 5-acetylamino-N,N'-Bis[2-hydroxy-1-(hydroxymethyl)ethyl]-5-[[(2S)-2-hydroxy-1-oxopropyl]amino]-2,4,6-triiodo-1,3-benzenedicarboxamide (hereinafter referred to as Impurity C): 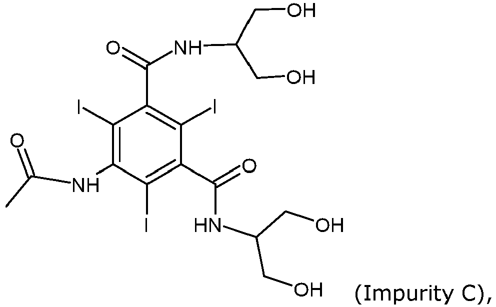 which is essentially ascribable to the presence of acetylating agents (i.e: acetic acid, acetyl chloride) as possible by-product formed in the presence of DMAC and (S)-2-(acetyloxy)propanoyl chloride. For instance, a precursor thereof is generated during the synthesis of intermediate (IV) by reacting the intermediate (III) with (S)-2-(acetyloxy)propanoyl chloride. The resulting impurity in the acyl chloride form is able to react in the subsequent step with serinol giving said Impurity C.

The current industrial work-up is unable to reduce its content to an acceptable limit, so the amount of this impurity must be under control during the reaction. Impurity C is currently classified in the group of the "total impurities" that should be kept below the maximum acceptable limit of 0.20 % w/w. Preferably, the amount of Impurity C must be ≤0.10 % w/w with respect to iopamidol.

Thus, there is still the need of a synthesis of iopamidol and the intermediates thereof that is reliable and provides excellent results in terms of yields and purity of the final product, while comprising a safer and efficient alternative to the use of DMAC and allowing the recovery and recycle of the reactants and solvents used, in order to decrease the waste production and save the costs of the industrial manufacturing connected to the waste treatment.

Surprisingly, it has now been found that the processes of the prior art, in particular the process described in WO2015/067601, can be carried out using a different, non-toxic solvent that also allows to obtain a more robust control on the formation of the Impurity C in the product, keeping it well below the acceptable threshold, at least below 0.08 % w/w. Moreover, it has been found that the overall process can be efficiently carried out using less amount of solvent.

The process of the invention was also found scalable and suitable for the industrial manufacturing of iopamidol.

### DESCRIPTION OF THE INVENTION

The present invention generally relates to a process for the industrial preparation of the non-ionic X-ray contrast agent S-(*N,N'*-bis[2-hydroxy-1-(hydroxymethyl)ethyl]-5-[[(2S)-2-hydroxy-1-oxopropyl]-amino]-2,4,6,triiodo-1,3-benzene dicarboxamide, commonly known as iopamidol, comprising novel conditions for the synthesis of this compound starting from 5-amino-N,N'-bis[2-hydroxy-1-(hydroxymethyl)ethyl]-2,4,6-triiodo-1,3-benzenedicarboxamide (IX) and providing an improved and more robust purity profile of the final product, fulfilling the Pharmacopoeia requirements.

It is therefore the object of the present invention the preparation of iopamidol comprising (a) the protection of the starting material 5-amino-N,N'-bis[2-hydroxy-1-(hydroxymethyl)ethyl]-2,4,6-triiodo-1,3-benzenedicarboxamide (IX) with a boric acid derivative thereof and (b) acylation of the corresponding protected intermediate (XI) with (S)-2-(acetyloxy)propanoyl chloride, followed by (c) the hydrolysis of the obtained protected derivative (XII), wherein steps a) and b) are carried out in a reaction medium comprising N-butyl pyrrolidone (NBP).

The process of the invention is advantageous with respect to the methods described in the prior art in that it is more robust and reliable in keeping under control the formation of the by-products such as Impurity C and it is more sustainable since it completely avoids the use of hazardous solvents, such as DMAC.

In particular, it was surprisingly found that, in accordance with the process of the present invention, the final product iopamidol can be obtained with a good efficiency, with yields higher than 84 %, and with a better purity profile, wherein the amount of Impurity C is controlled and kept below 0.08 % w/w, in addition without the use of toxic materials.

Accordingly, in one aspect the present invention relates to a process for the preparation of iopamidol comprising the following steps:
a) reacting 5-amino-*N,N'*-bis[2-hydroxy-1-(hydroxymethyl)ethyl]-2,4,6-triiodo-1,3-benzenedicarboxamide (IX) in a reaction medium with a boric acid derivative to obtain an intermediate of formula (XI), 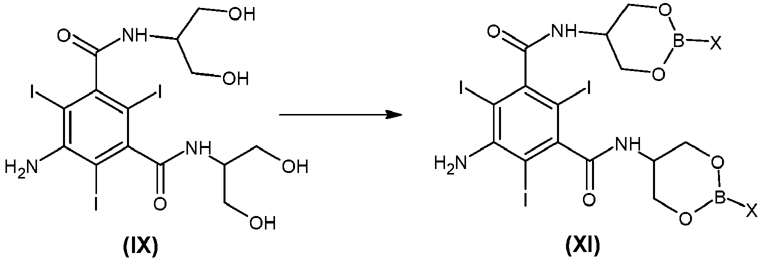 wherein X is selected from (C₁-C₆) alkyl, (C₃-C₆) cycloalkyl, (C₆) aryl, -O-(C₁-C₆) alkyl, -O-(C₃-C₆) cycloalkyl and -O-(C₆) aryl, optionally substituted with methyl, ethyl, n-propyl, i-propyl, n-butyl, sec-butyl, t-butyl and phenyl;
b) reacting said intermediate compound of formula (XI) with (S)-2-(acetyloxy)propanoyl chloride in a reaction medium and removing the boron protecting groups to provide the N-(S)-2-(acetyloxy)propanoyl compound of formula (XII) 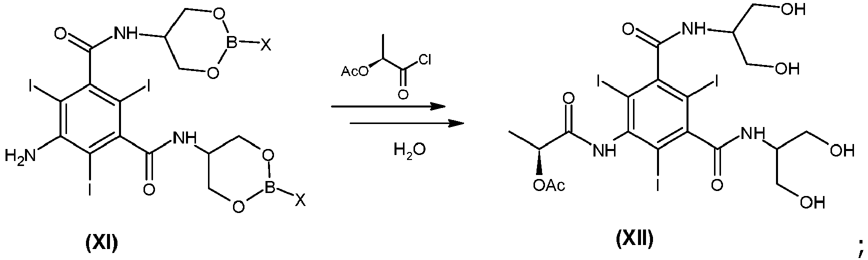
c) hydrolyzing the intermediate (XII) from step b) by adding a diluted alkaline solution to provide iopamidol 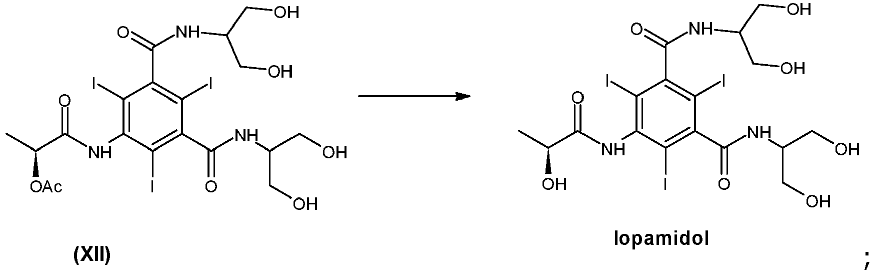 characterized in that the reaction medium of steps a) and b) is a mixture comprising N-butyl-pyrrolidone (NBP).

In one embodiment the compound of formula (IX) is protected with a boron derivative selected from the group consisting of 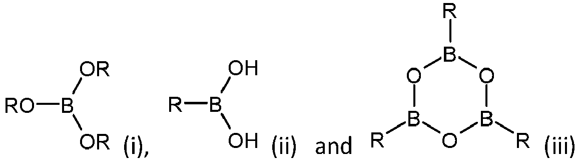 wherein R is a group selected from a (C₁-C₆) linear or branched alkyl, (C₃-C₆) cycloalkyl and (C₆) aryl, optionally substituted with a group selected from methyl, ethyl, n-propyl, i-propyl, n-butyl, sec-butyl, t-butyl and phenyl.

More preferably, the boric acid derivative is a boronic acid of formula (ii), as defined above, wherein R is a group (C₆) aryl. Even more preferably, R is a phenyl, optionally substituted with methyl.

Such derivatives used in the process of the present invention are well-known compounds commercially available or easily prepared according to literature methods, for instance as described in Boronic Acids, Edit. by D.G. Hall, 2011, Chapter 1: "Structure, Properties, and Preparation of Boronic Acid Derivatives. Overview of Their Reactions and Applications" and in US2003/0100792 A1.

Compounds of formula (i), for example, can be also obtained directly from reaction of boric acid with a suitable alcohol, as in the following general reaction: 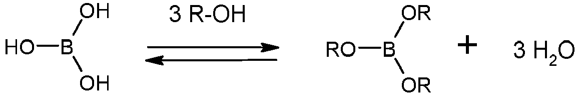 wherein R is as defined above.

In the latter case, the intermediate of formula (IX) can be also protected in step a) by treatment with boric acid and an R-OH alcohol in the reaction medium. Alternatively, the boric acid ester of formula (i) can be directly added.

In a most preferred embodiment, the compound of formula (IX) is reacted with a boronic acid selected from phenyl boronic acid, *p*-tolyl boronic acid, *m*-tolyl boronic acid and *o*-tolyl boronic acid. Even more preferably, the boric acid derivative is selected from *meta*-tolyl boronic acid and *para*-tolyl boronic acid.

In the present description, the expressions "(C₁-C₆) alkyl" or "(C₁-C₆) linear or branched alkyl" refer to an aliphatic hydrocarbon radical group, which may be a straight or branchedchain, having from 1 to 6 carbon atoms in the chain. Representative and preferred alkyl groups include methyl, ethyl, n-propyl, iso-propyl, n-butyl, tert-butyl, pentyl and hexyl.

The expression "(C₃-C₆) cycloalkyl" as used therein comprises within its meaning a saturated (i.e. cycloaliphatic) carbocyclic ring comprising from 3 to 6 carbon atoms. Suitable examples include a C₅-C₆ carbocyclic ring, e.g. a cyclohexyl ring.

The expression "(C₆) aryl" refers to a carbocyclic aromatic system containing six carbon atoms, such as phenyl. Such aromatic radical can be optionally substituted at any point of attachment of the aryl ring. Exemplary substituents include, but are not limited to, methyl, ethyl, n-propyl, iso-propyl, n-butyl and tert-butyl.

The expressions "boron derivative" or "boric acid derivative" generally comprise boron oxyacids, such as boronic acids, esters thereof and boroxine. In particular, it refers to the groups of formula (i), (ii) or (iii) as defined above.

In a preferred embodiment the process of the invention is a one-pot process, i.e. it is carried out without any purification steps of the intermediate and in particular without the isolation of the intermediates of formula (XI) and (XII) as defined above.

In one embodiment, the process of the invention is characterized in that the reaction medium of step a) is a mixture comprising *N*-butyl-pyrrolidone (NBP) and a co-solvent, which is preferably an organic water-immiscible solvent selected from the group consisting of 4-methyl-2-pentanone (MIBK), 3-pentanone, dibutyl ether, methyl tert-butyl ether, methyl ethyl ketone, methyl isopentyl ketone, ethyl acetate, butyl acetate and isopropyl acetate.

Preferably the reaction of step a) is carried out using 4-methyl-2-pentanone (MIBK) as co-solvent.

In a preferred embodiment the weight ratio between the co-solvent and NBP in the reaction medium of step a) is comprised between 1.2 and 0.5. Preferably it is 0.5.

Preferably, in steps a) and b) the weight ratio between NBP and compound (IX) is comprised between 1.2 and 2.5 w/w. More preferably it is 1.5 w/w.

In this respect, it was surprisingly found that the reactions of the invention can be efficiently performed even using less amounts of solvents compared with the methods described in the prior art. In fact, WO2015/067601 discloses in the examples that the reaction of protection with boric acid derivatives can be performed in DMAC as reaction medium, using a weight ratio between DMAC and the compound (IX) of 4 w/w. Conversely, it was found that in the process of the invention, using NBP (preferably in admixture with a co-solvent) instead of DMAC, it is no longer necessary to add amounts of reaction medium higher than 2.5 w/w with respect to compound (IX) as it is advantageously not necessary to have a highly diluted reaction medium.

In another embodiment the process described above is characterized in that the reaction medium of step b) consists of N-butyl-pyrrolidone (NBP) only.

It was also surprisingly found that, with respect to the process disclosed in WO2015/067601 (e.g. in the examples on pages 19-26), the acylation reaction of step b) can be efficiently performed even with an amount of (S)-2-(acetyloxy)propanoyl chloride lower than 2 equivalents, preferably comprised in the range from 1.6 to 2 equivalents with respect to compound (IX). More preferably, the amount of (S)-2-(acetyloxy)propanoyl chloride is 1.65 equivalents with respect to compound (IX).

Unexpectedly, it was observed that both the replacement of DMAC with NBP (with a lower amount of solvent used as reaction medium) together with the reduction of the amount of (S)-2-(acetyloxy)propanoyl chloride provided an improved purity profile of the final product iopamidol, because they both allowed to reduce the amount of Impurity C formed with the previously described methods.

The percentage of Impurity C obtained with the present process is shown for instance in Tables 1-2 of the experimental section, and in particular in Table 1 reporting the comparison with the value obtained using the process described in WO2015/067601.

According to the process of the invention, step a) can be carried out by loading compound (IX) in a reactor with the boron derivative and an admixture of NBP and a co-solvent, heating the suspension to a temperature comprised between 75 °C and 90 °C. Preferably, the temperature of step a) is comprised between 80 °C and 85 °C.

In particular, according to the invention the NBP solvent in step a) can be alternatively added at the beginning of the reaction together with the co-solvent or after a certain time where the co-solvent only is present.

The water formed as by-product during the reaction is preferably removed by distillation of the water/co-solvent mixture under vacuum, preferably at 450-350 mbar.

The distilled vapors can be condensed, collected and separated in two phases: preferably, the upper phase (organic phase) is constantly recirculated to the reactor, thus keeping constant the mixture volume. After concentration of the solution by distillation, NBP can be added and the solution distilled at a temperature comprised between 75 °C and 90 °C under vacuum (e.g. from 180 to 50 mbar).

In case of a one-pot process, the mixture can be preferably cooled at a temperature between 15 °C and 25 °C and, after a check to confirm the complete protection of the diol groups, be directly used as substrate for step b) without isolating the intermediate of formula (XI). Preferably, the process of the invention is carried out an one-pot process without isolating the intermediates.

According to step b) of the invention, the reagent (S)-2-(acetyloxy)propanoyl chloride can be added at a temperature between 20 °C and 25 °C to the solution obtained from step a). The obtained solution is then preferably heated to a temperature comprised between 20 °C and 40 °C to promote the reaction, then maintained under stirring until reaction completion, and cooled to a temperature comprised between 15 °C and 20 °C. More preferably, the acylation of step b) is carried out at a temperature comprised between 30 °C and 40 °C even more preferably at a temperature of 30 °C.

At the end of the reaction water is slowly added, to hydrolyse the boron-containing protective groups to the free hydroxyl groups, followed by a solution of 6.3% NaOH until pH of 6.0-7.0, keeping the temperature under 25 °C. After that, an organic immiscible solvent like MIBK can be added in the reactor, stirring for at least 5 minutes and then waiting until complete separation of the two phases. The lower aqueous phase can be transferred, and the upper organic phase is preferably back-extracted with water.

Optionally, the boron derivative used in step a) can be recovered from the organic phase together with the NBP solvent and re-used in a subsequent cycle of reactions. To reuse the organic phase containing the boronic acid the solution is distilled under vacuum (P 450-350 mbar, T= 80 °C) to remove the co-solvent excess (e.g. MIBK), then boronic acid content can be checked by HPLC analysis (see experimental section below) and optionally fresh boronic acid in solid form can be added to step a) of a subsequent batch process.

The aqueous phases containing compound (XII) can be put together to recover the product. Accordingly, the mixture collected from the aqueous phases after the solvent extraction can be conveniently treated with basic conditions (e.g. 30% NaOH) for a period ranging from 2 to 8 h (preferably 5 h), stirring at a temperature comprised between 30 °C and 40 °C. After cooling at room temperature and quenching with diluted HCl (e.g. 25 % HCl until pH of 6.5 -7.5) the mixture thus obtained can be purified in order to obtain the final iopamidol.

The process of the invention can be conveniently followed by a chromatographic purification on a conventional column comprising a solid phase selected from microporous highly cross-linked styrene resins, preferably Amberlite^{®} XAD 1600, 1600 T and 16 (Rohm & Haas) or equivalents marketed by other manufacturers. The elution can be carried out with water and diluted NaOH, washing until disappearance of the compound from the resin.

After concentration of the aqueous phase, desalting is preferably performed by nanofiltration, followed by an ionic exchange step with a battery (in series or in mixed bed) consisting of a strong cationic resin of sulfonic type, regenerated in acidic form and a mean anionic resin of a secondary amine type, regenerated in the OH-form, for instance following a known procedure already described in WO97/30735.

In a preferred embodiment, the cationic resins are selected from the group consisting of: Dowex 350, Amberjet 1200 and Amberlite IR120, preferably Dowex 350. The preferred anionic resin is Relite MG1P.

CARBOPURON 4N can be added to the final solution and the suspension heated to 55 °C. After 1 h, the suspension is filtered. The resulting desalted solution can be concentrated and purified by crystallization from a suitable solvent, as already known in literature (e.g. described in GB 1,472,050).

Advantageously, it was found that working in the absence of the dipolar aprotic solvent DMAC, always present in the processes of the prior art, the work-up of step c) can be carried out in a simplified manner reducing the volume resins, the total reaction time and consequently the final collected volume containing iopamidol. Moreover, the present process also allows to dramatically reduce the amounts of waste solutions with respect to the processes of the prior art. In fact, the chromatographic work-up after step c) in the process of the invention surprisingly allows to reduce the final waste volumes up to 80 % with respect to the process described in WO2015/067601.

The process of the invention is more robust and reliable, and it allows to obtain iopamidol with a content of Impurity C not higher than 0.08 % w/w, or preferably not higher than 0.05%, below the acceptable specification limit (≤ 0.10 % w/w).

No NBP from the preparation steps a) and b), neither other residual solvent (from the above or other synthetic steps), were detected in the final product in amounts higher than the requirements established by ICH (International Conference on Harmonization) concerning the presence of residual solvents in pharmaceuticals.

Further details concerning the manufacturing of iopamidol according to the present invention are reported in the following experimental section, with the sole aim to better illustrate the present invention, without representing any limitation to it.

### EXPERIMENTAL SECTION

### Materials and methods

All commercially available reagents and solvents were used without further purification. The intermediate (IX) was prepares according to known methods, for instance as described in WO2015/067601.

Reaction products and intermediates, as per the following examples, were analyzed by HPLC techniques, for instance using the conditions as follows:

| | |
|---|---|
| Column: | Two Zorbax SB-Phenyl 4.6x250 mm 5 µm |
| Temperature: | 60 °C |
| Mobile phase: | A: H₂O; B: H₂O/ACN 1/1, 50/50 gradient elution |
| Detection (UV): | 225 and 240 nm |

The data obtained by HPLC analysis are reported in area %.

### List of abbreviations:

NBP: *N*-butyl-pyrrolidone
DMAC: dimethylacetamide
MIBK: methyl-isobutyl-ketone
PBA: phenyl boronic acid
m-TBA: *meta*-tolyl boronic acid
p-TBA: *para*-tolyl boronic acid
HPLC: high performance liquid chromatography
rT: room temperature

### Example 1

### One-pot synthesis of iopamidol starting from 5-amino-N,N'-bis[2-hydroxy-1-(hydroxymethyl)ethyl]-2,4,6-triiodo-1,3-benzenedicarboxamide (IX) and using phenyl boronic acid (PBA) as protecting group.

Phenyl boronic acid (PBA) (31.68 g; 0.303 mol), compound (IX) (100.23 g; 0.142 mol), and methyl isobutyl ketone (MIBK) (300.53 g) were loaded in a 500 mL reactor at rT. The suspension obtained was heated to 81 °C and a water/MIBK mixture was distilled under vacuum (380 mbar) for 2.5 h, in order to remove the water formed as by-product during the reaction (about 10.22 g). The distilled vapours were condensed, collected in a decanter and separated into two liquid phases: the upper phase (organic phase) was constantly recirculated to the reactor, keeping constant the mixture volume. The suspension was concentrated by distillation of about 150 g of water/MIBK, then *N*-butyl-2-pyrrolidone (NBP) (210,00 g) was added to obtain a solution. This solution was distilled at 83 °C under vacuum (50 mbar), until collecting an overall amount of distillate of about 300 g. The mixture in reactor was checked to confirm that the protection was completed.

After cooling at 20 °C, (S)-2-(acetyloxy)propanoyl chloride (38.58 g; 0.256 mol; 1.8 eq) was added to the solution in about 1.5 h, maintaining the temperature at 20 °C. The obtained solution was heated to 26 °C and maintained under stirring until the condensation was completed (about 39 h). Then the mixture was cooled to 15 °C and water (180.68 g) was slowly added keeping the temperature under 25 °C.

After that, a solution of 6.3% NaOH (about 238.74 g), previously prepared, was added until pH 6.48 keeping the temperature under 25 °C.

Crude iopamidol: 94.5% (area % HPLC); conversion yield: 99%.

Impurity C: 0.042 % w/w (with respect to iopamidol).

### Example 2

### One-pot synthesis of iopamidol using m-tolyl boronic acid (m-TBA) as protecting group and 2 equivalents of (S)-2-(acetyloxy)propanoyl chloride

m-TBA (35.84 g 0.298 mol), compound (IX) (100.02 g; 0.142 mol), MIBK (300.14 g) and NBP (100.08 g) were loaded in a 500 mL reactor at rT. The suspension obtained was heated to 82 °C and a water/MIBK mixture was distilled under vacuum (320 mbar) for 2.75 h, in order to remove the water formed as by-product during the reaction (about 10.22 g). During the distillation the suspension become a solution. The distilled vapours were condensed, collected in a decanter and separated into two liquid phases: the upper phase (organic phase) was constantly recirculated to the reactor, keeping constant the mixture volume.

Then the suspension was concentrated by distillation of about 150 g of water/MIBK, then NBP (150.81 g) was added. This solution was distilled at 85 °C under vacuum (60 mbar), until collecting an overall amount of distillate of about 300 g. The mixture in reactor was checked to confirm that the protection was completed. After cooling at 20 °C, (S)-2-(acetyloxy)propanoyl chloride (42.71 g; 0.284 mol; 2.0 eq) was added to the solution in about 0.5 h, maintaining the temperature between 20 ÷ 25 °C.

The obtained solution was heated to 40 °C and maintained under stirring until the condensation was completed (about 23 h). Then the mixture was cooled to 15 °C and water (181.60 g) is slowly added keeping the temperature under 25 °C. After that, a solution of 6.3% NaOH (about 267.9 g), previously prepared, was added until pH 6.76 keeping the temperature under 25 °C. MIBK (259.85 g) was added to the solution contained in the reactor, the two phases were stirred for at least 5 minutes; then was waited about 10 minutes until complete separation. The lower aqueous phase was transferred, and the upper organic phase was back-extracted with water (200.08 g).

The aqueous phases were put together (iopamidol recovered in aqueous phase was 86.33%)
Crude iopamidol: 95.0% (area% HPLC)
Impurity C: 0.043% w/w (with respect to iopamidol).

### Example 3

### One-pot synthesis of iopamidol using m-tolyl boronic acid (m-TBA) as protecting group and 1.8 equivalents of (S)-2-(acetyloxy)propanoyl chloride

m-TBA (35.88 g 0.298 mol), compound (IX) (100.14 g; 0.142 mol), MIBK (300.24 g) and NBP (100.28 g) were loaded in a 500 mL reactor at rT. The suspension obtained was heated to 83 °C and a water/MIBK mixture was distilled under vacuum (320 mbar) for 2.75 h, in order to remove the water formed as by-product during the reaction (about 10.22 g). During the distillation the suspension become a solution. The distilled vapours were condensed, collected in a decanter and separated into two liquid phases: the upper phase (organic phase) was constantly recirculated to the reactor, keeping constant the mixture volume.

Then the suspension was concentrated by distillation of about 150 g of water/MIBK, then NBP (100.21 g) was added. This solution was distilled at 85 °C under vacuum (60 mbar), until collecting an overall amount of distillate of about 300 g. The mixture in reactor was checked to confirm that the protection is completed. After cooling at 20 °C, (S)-2-(acetyloxy)propanoyl chloride (38.47 g; 0.256 mol; 1.8 eq) was added to the solution in about 0.5 h, maintaining the temperature between 20 ÷ 25 °C.

The obtained solution was heated to 30 °C and maintained under stirring until the condensation was completed (about 41 h). Then the mixture was cooled to 15 °C and water (180.39 g) was slowly added keeping the temperature under 25 °C. After that, a solution of 6.3% NaOH (about 236.55 g), previously prepared, was added until pH = 6.20 keeping the temperature under 25 °C

Crude iopamidol 96.5 % (area% HPLC); conversion yield: 99%.

Impurity C: 0.038 % w/w (with respect to iopamidol).

### Example 4

### One-pot synthesis of iopamidol using m-tolyl boronic acid (m-TBA) as protecting group and 1.6 equivalents of (S)-2-(acetyloxy)propanoyl chloride

m-TBA (35.98 g 0.299 mol), compound (IX) (100.22 g; 0.142 mol), MIBK (300.38g) and NBP (100.24 g) were loaded in a 500 mL reactor at rT. The suspension obtained was heated to 82 °C and a water/MIBK mixture was distilled under vacuum (320 mbar) for 3 h, in order to remove the water formed as by-product during the reaction (about 10.22 g). During the distillation the suspension become a solution. The distilled vapours were condensed, collected in a decanter and separated into two liquid phases: the upper phase (organic phase) is constantly recirculated to the reactor, keeping constant the mixture volume.

Then the suspension is concentrated by distillation of about 150 g of water/MIBK, then NBP (50.04 g) is added. This solution is distilled at 85 °C under vacuum (60 mbar), until collecting an overall amount of distillate of about 300 g. The mixture in reactor is checked to confirm that the protection is completed. After cooling at 20 °C, (S)-2-(acetyloxy)propanoyl chloride (34.20 g; 0.227 mol; 1.6 eq) was added to the solution in about 0.25 h, maintaining the temperature between 20 °C.

The obtained solution was heated to 20 °C and maintained under stirring until the condensation was completed (about 41 h). Then the mixture is cooled to 15 °C and water (180.84 g) is slowly added keeping the temperature under 25 °C. After that, a solution of 6.3% NaOH (about 235.15 g), previously prepared, was added until pH= 6.09 keeping the temperature under 25 °C.

Crude iopamidol: 94.2 % (area % HPLC); conversion yield: 99 %.

Impurity C: 0.026 % w/w (with respect to iopamidol).

### Example 5

### One-pot synthesis of iopamidol using m-tolyl boronic acid (m-TBA) as protecting agent and 1.65 eq. of (S)-2-(acetyloxy)propanoyl chloride (4X scale)

m-TBA (140.81 g 1.193 mol), compound (IX) (400.22 g; 0.568 mol), MIBK (1198.17 g) and NBP (399.64 g) were loaded in a 4 L reactor at rT. The suspension obtained is heated to 83 °C and a water/MIBK mixture was distilled under vacuum (320 mbar) for 3 h, in order to remove the water formed as by-product during the reaction (about 40.89 g). During the distillation the suspension become a solution. The distilled vapours were condensed, collected in a decanter and separated into two liquid phases: the upper phase (organic phase) was constantly recirculated to the reactor, keeping constant the mixture volume.

Then the suspension was concentrated by distillation of about 668.40 g of water/MIBK, then NBP (200.13 g) was added. This solution was distilled at 85 °C under vacuum (60 mbar), until collecting an overall amount of distillate of 1165.05 g. The mixture in reactor was checked to confirm that the protection was completed.

After cooling at 20 °C, (S)-2-(acetyloxy)propanoyl chloride (141.25 g; 0.938 mol; 1.65 eq) was added to the solution in about 1.5 h, maintaining the temperature at 20 °C.

The obtained solution was heated to 32 °C and maintained under stirring until the condensation was completed (about 24 h). Then the mixture is cooled to 15 °C and water (710.38 g) was slowly added keeping the temperature under 25 °C. After that, a solution of 6.3 % NaOH (about 846.61 g), previously prepared, was added until pH =6.3 keeping the temperature under 25 °C.

Crude iopamidol: 94.4 % (area % HPLC); conversion yield: 99 %.

Impurity C: 0.048 % w/w (with respect to iopamidol).

In order to remove the m-TBA and NBP after step a), the quenched mixture was extracted with MIBK at rT, following the procedure reported below:
MIBK (1040.33g) was added to the aqueous solution contained in the reactor, the two phases were stirred for at least 5 minutes; then the stirring was stopped and about 15 minutes waited until complete separation. The lower aqueous phase was transferred.

After the extraction, the organic phase was back-extracted with water (3 x 400 g); the lower aqueous phases were added to the previous aqueous solution. The final aqueous phase was extracted 2 times with MIBK, and then was heated to 50 °C to remove MIBK traces by distillation, under reduce pressure (110 mbar).

The solution was cooled to 35 °C; and 30 % NaOH (189.18 g; 1.419 mol; 2.5 eq) was added in 15 min. The mixture was stirred at this temperature for 5 h; then was cooled at room temperature; 25 % aqueous HCl (70.15 g 0.481 0.85 eq) was added to reach pH= 7.26.

### Example 6

### One-pot synthesis of iopamidol using a recycled solution of m-tolyl boronic acid (m-TBA) in NBP

The organic phase (967.05 g) of a previous reaction was heated at 80 °C and MIBK was distilled under vacum (350 mbar) until reach the desired concentration (residue 412.88 g).

Then compound (IX) (100.08 g, 0.142 mol), m-TBA (2.40 g, 0.020 mol) were added. The suspension obtained was heated to 83 °C and a water/MIBK mixture was distilled under vacuum (320 mbar) for 3 h, in order to remove the water formed as by-product during the reaction (about 17.03 g). During the distillation the suspension become a solution. The distilled vapours were condensed, collected in a decanter and separated into two liquid phases: the upper phase (organic phase) was constantly recirculated to the reactor, keeping constant the mixture volume.

Then the suspension was concentrated by distillation of about 150 g of water/MIBK, then NBP (20.37 g) was added. This solution was distilled at 85 °C under vacuum (60 mbar), until collecting an overall amount of distillate of about 300 g. The mixture in reactor was checked to confirm that the protection was completed. After cooling at 20 °C (S)-2-(acetyloxy)propanoyl chloride (35.54 g; 0.236 mol; 1.66 eq) was added to the solution in about 0.5 h, maintaining the temperature at 20 °C. The obtained solution was heated to 32 °C and maintained under stirring until the condensation was completed (23 h). Then the mixture was cooled to 15 °C and water (180.10 g) was slowly added keeping the temperature under 25 °C. After that, a solution of 6.3% NaOH (about 208.26 g), previously prepared, was added until pH = 6.17 keeping the temperature under 25 °C.

Iopamidol: 93.8 % (area % HPLC); conversion: about 99%.

Impurity C: 0.069 % w/w (with respect to iopamidol).

### Example 7

### Final Purification and crystallization of iopamidol

### Purification of the mixture obtained from Example 5 (work-up after step c)):

The mixture derived from Example 5 (2952 g) was eluted (flow rate = 2.15 BV/h) on a Amberlite XAD column (856 mL); then the column was washed with water (1290 g, flow rate 2.5 BV/h) and with 0.025 % aqueous NaOH (3490 g flow rate 2.5 BV/h).

The solution was fed to the nanofiltration plant; in a first step the solution was concentrated to 30% w/w of iopamidol, keeping the pH between 5.5 and 6.5 by addition of HCl or NaOH. Then the diafiltration was continued, keeping constant the volume by addition of water, until the retentate conductivity was lower than 10 mS/cm.

The retentate was send to three in series exchange resin columns (C1: strong cationic Dowex C350 - 568 mL; C2: weak anionic Relite MG1/P - 640 mL; C3: strong cationic Dowex C350 - 64 mL), at rT and at 1.8 BV/h (referred to C2). After loading, the column series was washed with water (2051 g).

The solution (5598.50 g) was collected in a reactor for concentration at 50 °C under vacuum (Pressure: 110 ÷100 mbar), until iopamidol concentration of about 18% w/w was reached; CARBOPURON 4N (6.63 g) was added and the suspension was heated to 55 ÷60 °C. After approximately 1 h, the suspension was filtered on a 0.2 µm filter.

The solution was further distilled at 50 ÷ 55 °C under vacuum (P 110 ÷ 80 mbar) until a concentration of iopamidol of about 50 % w/w (the amount of distillate collected was 1649.82 g). 2-butanol (765.57 g) was added to the mixture was heated to 92 °C. The 2-butanol/water mixture was distilled at atmospheric pressure, keeping the level constant by addition of the same weight of fresh 2-butanol, until KF= 3.90 %.

The obtained suspension was stirred a reflux for additional 15 min, cooled to a temperature below 25 °C and filtered. The cake was washed with 2-butanol (2 x 400 g). The wet solid (428.03 g) was dried at 60 °C under vacuum (P = 20 mbar) for 16 h.

371.42 g of dried iopamidol was obtained.

Iopamidol final yield: 84.2%.

### Example 8

### Comparison data relating to Impurity C

The results relating to the amount of Impurity C (% w/w with respect to iopamidol) obtained using the process of the present invention were compared with those obtained reproducing the process described in WO2015/067601. Table 1 shows the % amount of the Impurity C obtained in the conditions of the present invention (average value from at least three batches), i.e. using the solvent NBP with a weight ratio NBP/compound (IX) of 1.5, and in the conditions described in WO2015/067601, i.e using the solvent DMAC with a weight ratio DMAC/compound (IX) of 1.9. In both cases, step b) was carried out at 30 °C using 1.8 equivalents of (S)-2-(acetyloxy)propanoyl chloride with respect to compound (IX).

**Table 1**

| | **Impurity C (w/w)** |
|---|---|
| Process of the invention | 0.039 % |
| Process described in WO2015/067601 (Ref.) | 0.098 % |

From these results, it can be noted that the process of the present invention, besides having the advantage of avoiding the use of toxic solvents and reducing in general the amount of solvent necessary to perform the reaction steps, it is also surprisingly able to reduce the amount of Impurity C formed during the reaction of diol protection.

Using the conditions of other preferred embodiments of the present invention, it is possible to further reduce the content of Impurity C, as it is illustrated below. In fact, the following Table 2 reports the amounts of Impurity C obtained with a weight ratio of NBP/compound (IX) of 1.5 in step a) and different conditions of temperature and equivalents of (S)-2-(acetyloxy)propanoyl chloride for step b). The results of experiments 1 and 6 corresponds respectively to the Examples 4 and 6 described above, while experiments 2-5 were carried out in an analogous way simply changing the parameters highlighted below:

**Table 2**

| Experiment # | Weight ratio NBP/comp. (IX) | Equivalents of (S)-2-(acetyloxy)propanoyl chloride | Temper. | **Impurity C (w/w)** |
|---|---|---|---|---|
| 1 (Example 4) | 1.5 | 1.6 | 20 °C | 0.026 % |
| 2 | 1.5 | 2.0 | 20 °C | 0.027 % |
| 3 | 1.5 | 1.8 | 30 °C | 0.039 % |
| 4 | 1.5 | 1.6 | 40 °C | 0.079 % |
| 5 | 1.5 | 2.0 | 40 °C | 0.076 % |
| 6 (Example 2) | 2.5 | 2.0 | 40 °C | 0.043 % |

### Example 9

### Comparison data relating to the waste volumes after step c) of the invention.

The following Table 3 shows a comparison of the final collected volumes of the solution containing desalted iopamidol obtained after the chromatographic work-up of the present process (see Example 7) compared to those obtained with the conditions described in WO2015/067601.

For comparison, the volumes are calculated with reference to 1 kg of compound (IX).

**Table 3**

| Ion exchange resin | Process of the invention | Process described in WO2015/067601 (Ref.) |
|---|---|---|
| Dowex C350 | 1.6 L | 3.2 L |
| Relite MG-1P | 1.6 L | 3.4 L |
| Diaion PA308 | - | 5.0 L |
| **Total volumes** | **3.2 L** | **11.6 L** |

From these results, it can be noted that the process of the present invention also provides the advantage of reducing of the amount of final waste derived from the chromatographic process up to 80 %.

### References:

1. The Merck Index, RSC Publishing, 15th Ed., 2013, 940-941;
2. Lusic, H. et al., Chem. Rev. 2013, 113, 1641-1666
3. GB 1,472,050
4. US 4,001,323
5. WO2002/044125
6. WO2015/067601
7. Boronic Acids, Edit. by D.G. Hall, 2011, Chapter 1
8. US2003/0100792 A1
9. WO97/30735

## Claims

1. A process for the preparation of iopamidol (II) comprising the following steps:
a) reacting 5-amino-*N,N'*-bis[2-hydroxy-1-(hydroxymethyl)ethyl]-2,4,6-triiodo-1,3-benzenedicarboxamide (IX) in a reaction medium with a boric acid derivative thereof to obtain an intermediate of formula (XI), 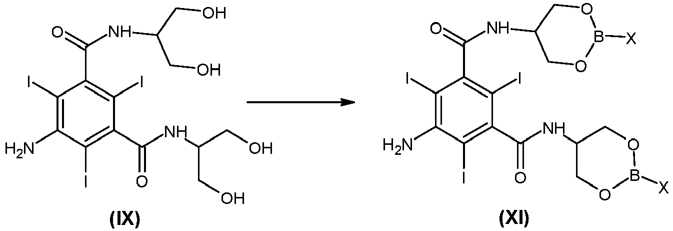 wherein X is selected from (C₁-C₆) alkyl, (C₃-C₆) cycloalkyl, (C₆) aryl, -O-(C₁-C₆) alkyl, -O-(C₃-C₆) cycloalkyl and -O-(C₆) aryl, optionally substituted with methyl, ethyl, n-propyl, i-propyl, n-butyl, sec-butyl, t-butyl and phenyl;
b) reacting said intermediate compound of formula (XI) with (S)-2-(acetyloxy)propanoyl chloride in a reaction medium and removing the boron protecting groups to provide the N-(S)-2-(acetyloxy)propanoyl compound of formula (XII) 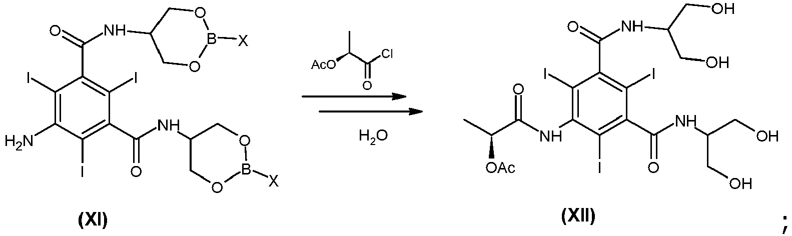
c) hydrolyzing the intermediate (XII) from step b) by adding a diluted alkaline solution to provide iopamidol 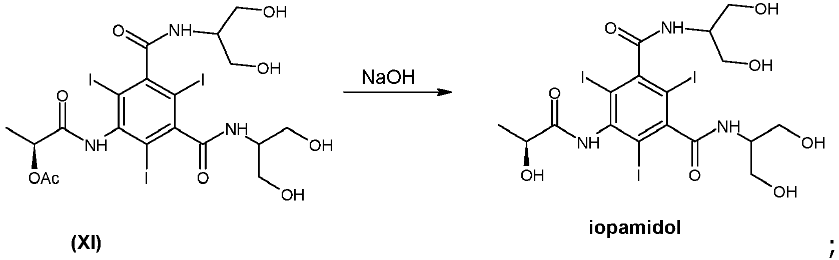 **characterized in that** the reaction medium of steps a) and b) is a mixture comprising *N-*butyl-pyrrolidone (NBP).

2. The process of claim 1 **characterized in that** the reaction medium of step a) is a mixture comprising *N*-butyl-pyrrolidone (NBP) and a co-solvent selected from the group consisting of 4-methyl-2-pentanone (MIBK), 3-pentanone, dibutyl ether, methyl tert-butyl ether, methyl ethyl ketone, methyl isopentyl ketone, ethyl acetate, butyl acetate and isopropyl acetate.

3. The process according to claim 2 wherein the weight ratio between the co-solvent and NBP in the reaction medium of step a) is comprised between 1.2 and 0.5.

4. The process of claim 1 **characterized in that** the reaction medium of step b) consists of *N-*butyl-pyrrolidone.

5. The process of claim 1 **characterized in that** the boric acid derivative is selected from the group consisting of 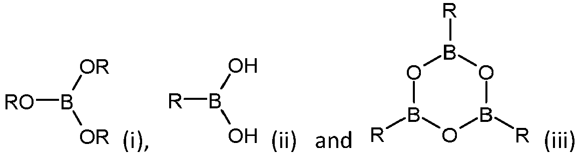 wherein R is a group selected from a (C₁-C₆) linear or branched alkyl, (C₃-C₆) cycloalkyl and (C₆) aryl, optionally substituted with a group selected from methyl, ethyl, n-propyl, i-propyl, n-butyl, sec-butyl, t-butyl and phenyl.

6. The process according to claim 5 wherein the boric acid derivative is a compound of formula (ii), wherein R is a group (C₆) aryl, optionally substituted with methyl.

7. The process according to claim 6 wherein the boric acid derivative is selected from *meta-*tolyl boronic acid and *para*-tolyl boronic acid.

8. The process according to claim 1 wherein in steps a) and b) the weight ratio between NBP and compound (IX) is comprised between 1.2 and 2.5 w/w.

9. The process according to claim 8, wherein the weight ratio between NBP and compound (IX) is 1.5 w/w.

10. The process according to claim 1, wherein in step b) the amount of (S)-2-(acetyloxy)propanoyl chloride is comprised between 1.6 and 2 equivalents with respect to compound (IX).

11. The process according to claim 10, wherein in step b) the amount of (S)-2-(acetyloxy)propanoyl chloride is 1.65 equivalents with respect to compound (IX).

12. The process according to claim 1, wherein step b) is carried out at a temperature comprised between 20 °C and 40 °C.

13. The process according to claim 12, wherein step b) is carried out at a temperature of 30 °C.

14. The process according to anyone of claims 1-13, which is a one-pot process.

15. The process according to claim 1, wherein the boric acid derivative used for diol protection in step a) is recovered in a mixture with NBP and recycled.
